# EUROPÄISCHE PATENTSCHRIFT

(11) **EP 0 877 741 B1**
(45) Veröffentlichungstag und Bekanntmachung des Hinweises auf die Patenterteilung: **23.07.2003**
(21) Anmeldenummer: 97900951.1
(22) Anmeldetag: 03.01.1997
(51) Int. Cl.: C07D 285/08, A01N 43/82

(54) **ACYLIERTE 5-AMINO-1,2,4-THIADIAZOLE ALS PESTIZIDE UND FUNGIZIDE**
ACYLATED 5-AMINO-1,2,4-THIADIAZOLES AS PESTICIDES AND FUNGICIDES
5-AMINO-1,2,4-THIADIAZOLES ACYLES UTILISES COMME PESTICIDES ET FONGICIDES

(30) Priorität: 15.01.1996 DE 19601139
(43) Veröffentlichungstag der Anmeldung: 18.11.1998
(73) Patentinhaber: Bayer CropScience AG, 40789 Monheim (DE)
(72) Erfinder: HEIL, Markus, D-51381 Leverkusen (DE); ERDELEN, Christoph, D-42799 Leichlingen (DE); WACHENDORFF-NEUMANN, Ulrike, D-56566 Neuwied (DE); TURBERG, Andreas, D-40699 Erkrath (DE); MENCKE, Norbert, D-51381 Leverkusen (DE); HÄNSSLER, Gerd, D-51381 Leverkusen (DE); STENZEL, Klaus, D-40595 Düsseldorf (DE)
(86) Internationale Anmeldenummer: EP9700012
(87) Internationale Veröffentlichungsnummer: WO97026251

(56) Entgegenhaltungen:
- EP-A- 0 378 308
- EP-A- 0 389 901
- EP-A- 0 623 282
- DE-A- 3 505 432

## Beschreibung

Die vorliegende Erfindung betrifft neue acylierte 5-Amino-1,2,4-thiadiazole, ein Verfahren zur ihrer Herstellung und ihre Verwendung zur Bekämpfung von tierischen Schädlingen und als Fungizide.

Es ist bereits bekannt, daß bestimmte acylierte 4-Cyano-5-aminoisothiazole insektizide Eigenschaften aufweisen (vgl. z.B. EP-A-0 623 282).

Die Wirksamkeit und Wirkungsbreite dieser Verbindungen ist jedoch insbesondere bei niedrigen Aufwandmengen und Konzentrationen nicht immer voll zufriedenstellend.

Es wurden neue acylierte 5-Amino-1,2,4-thiadiazole der Formel (I) gefunden, 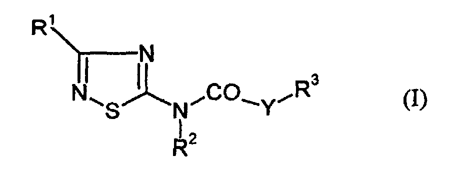 in welcher
- R¹: für C₁-C₄-Alkyl, C₁-C₄-Halogenalkyl mit 1 bis 5 gleichen oder verschiedenen Halogenatomen, wie Fluor-, Chlor- und Bromatomen, C₁-C₄-Alkoxy-C₁-C₄alkyl, C₁-C₄-Alkylthio-C₁-C₄-alkyl, C₁-C₄-Alkoxy, C₁-C₄-Alkylthio oder fiir gegebenenfalls einfach bis dreifach, gleich oder verschieden durch C₁-C₄-Alkyl oder Halogen substituiertes C₃-C₆-Cycloalkyl steht,
- R²: für Wasserstoff, C₁-C₄-Alkyl, C₁-C₄-Halogenalkyl mit 1 bis 5 gleichen oder verschiedenen Halogenatomen, wie Fluor- und Chloratomen, C₁-C₄-Alkoxy-C₁-C₄-alkyl, C₁-C₄-Alkyl-carbonyl, C₁-C₄-Alkylsulfonyl, jeweils gegebenenfalls einfach bis dreifach, gleich oder verschieden im Phenylring substituiertes Phenylcarbonyl, Phenylsulfonyl oder Benzyl steht, wobei als Substituenten jeweils Halogen, Nitro, Cyano, C₁-C₄-Alkyl, C₁-C₂-Halogenalkyl mit 1 bis 5 gleichen oder verschiedenen Halogenatomen, wie Fluor- und Chloratome, C₁-C₄-Alkoxy, C₁-C₄-Alkylthio, C₁-C₂-Halogenalkoxy mit 1 bis 5 gleichen oder verschiedenen Halogenatomen, wie Fluor- und Chloratome oder C₁-C₂-Halogenalkylthio mit 1 bis 5 gleichen oder verschiedenen Halogenatomen, wie Fluor- und Chloratome in Frage kommen, oder für gegebenenfalls einfach bis dreifach, gleich oder verschieden durch C₁-C₄-Alkyl oder Halogen substituiertes C₃-C₆-Cycloalkyl steht,
- R³: für gegebenenfalls einfach bis dreifach, gleich oder verschieden substituiertes Phenyl steht, wobei als Substituenten Halogen, Nitro, Cyano, C₁-C₁₂-Alkyl, C₁-C₁₂-Halogenalkylmit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₆-Cycloalkyl sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratome oder C₁-C₄-Alkoxy-carbonyl substituiertes Phenyl, Phenoxy, Phenylthio oder Benzyl in Frage kommen, oder für jeweils gegebenenfalls einfach bis dreifach, gleich oder verschieden substituiertes C₃-C₈-Cycloalkyl oder C₅-C₈-Cycloalkenyl steht, wobei als Substituenten C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₃-C₈-Cycloalkyl, sowie gegebenenfalls einfach bis dreifach, gleich oder verschieden durch Halogen, Cyano, Nitro, C₁-C₄-Alkyl, C₁-C₄-Halogenalkyl mit 1 bis 5 gleichen oder verschiedenen Halogenatomen, wie Fluor- und Choratome, C₁-C₄-Alkoxy oder C₁-C₄-Halogenalkoxy mit 1 bis 5 gleichen oder verschiedenen Halogenatomen, wie Fluor- und Chloratome, substituiertes Phenyl in Frage kommen und
- Y: für C₁-C₆-Alkylen, C₁-C₆-Hydroxyalkylen, C₁-C₄-Alkoxy-C₁-C₆-alkylen, C₁-C₄-Alkylcarbonyloxy-C₁-C₆-alkylen, Cyano-C₁-C₆-alkylen, C₁-C₄-Halogenalkylen mit 1 bis 5 gleichen oder verschiedenen Halogenatomen, wie Fluorund Chloratome; gegebenenfalls einfach bis dreifach, gleich oder verschieden durch Fluor, Chlor oder Methyl substituiertes C₃-C₆-Cycloalkyl-C₁-C₄alkylen, C₂-C₄-Alkenylen oder C₁-C₄-Alkylenoxy steht.

Weiterhin wurde gefunden, daß man die acylierten 5-Amino-1,2,4-thiadiazole der Formel (I) erhält, wenn man
5-Amino-1,2,4-thiadiazole der Formel (II) 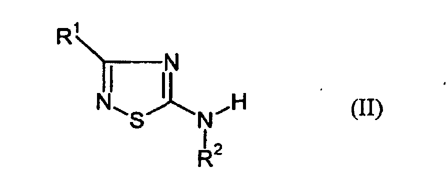 in welcher
- R¹ und R²: die oben angegebene Bedeutung haben,
mit Säurehalogeniden der Formel (III)

Hal―CO―Y―R³ (III)

in welcher
- R³ und Y: die oben angegebene Bedeutung haben und
- Hal: für Halogen steht,
in Gegenwart einer Base und in Gegenwart eines Verdünnungsmittels umsetzt.

Schließlich wurde gefunden, daß die neuen acylierten 5-Amino-1,2,4-thiadiazole der Formel (I) stark ausgeprägte biologische Eigenschaften besitzen und vor allem als Fungizide und zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen, geeignet sind.

Die erfindungsgemäßen acylierten 5-Amino-1,2,4-thiadiazole sind durch die Formel (I) allgemein definiert.

Bevorzugte Substituenten bzw. Bereiche der in den oben und nachstehend erwähnten Formeln aufgeführten Reste werden im folgenden erläutert.
- R¹: steht bevorzugt für Methyl, Ethyl, n- oder i-Propyl, n-, i-, s- oder t-Butyl; CH₂F, CHF₂, CF₃, CH₂Cl, CH₂Br, CHClCH₃; Methoxy, Ethoxy, Methoxymethyl, Ethoxymethyl; Methylthiomethyl oder Cyclopropyl.
- R²: steht bevorzugt fiir Wasserstoff, Methyl, Ethyl, n- oder i-Propyl; Methoxymethyl, Ethoxymethyl, n-Propoxymethyl, n-Butoxymethyl; Methylcarbonyl, Methylsulfonyl; jeweils gegebenenfalls einfach oder zweifach, gleich oder verschieden durch Fluor, Chlor, Methyl oder Trifluormethyl substituiertes Phenylcarbonyl oder Benzyl; oder Cyclopropyl.
- R³: steht bevorzugt für gegebenenfalls einfach bis dreifach, gleich oder verschieden substituiertes Phenyl, wobei als Substituenten Halogen, Nitro, Cyano, C₁-C₁₂-Alkyl, C₁-C₁₂-Halogenalkylmit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- und Chloratomen, C₂-C₄-Alkenyl, C₂-C₄-Halogenalkenyl mit 1 bis 5 gleichen oder verschiedenen Halogenatomen, wie Fluor- und Chloratomen, sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- oder Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratome oder C₁-C₄-Alkoxy-carbonyl substituiertes Phenoxy oder Phenylthio in Frage kommen.
- Y: steht bevorzugt für eine der Gruppen
-CH₂-, -CH(CH₃)-, -CH(C₂H₅)-, -CH(n-C₃H₇)-, -CH(i-C₃H₇)-, -CH₂CH₂-, -CH(OH)-, -CH(OCH₃)-, -CH(O-CO-CCH₃)-, -CH(CN)-, -CHF-, -CHCI-, -CH-[ 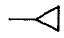 ]-, -CH=CH- oder -CH₂O-.
- R¹: steht besonders bevorzugt für Methyl, Ethyl, n- oder i-Propyl, n-, i-, s- oder t-Butyl; CH₂F, CHF₂, CF₃, CH₂Cl, CH₂Br, Methoxy, Ethoxy oder Cyclopropyl.
- R²: steht besonders bevorzugt für Wasserstoff, Methyl, Ethyl, Methoxymethyl, Ethoxymethyl, Methylcarbonyl, Phenylcarbonyl oder Methylsulfonyl.
- R³: steht besonders bevorzugt für gegebenenfalls einfach bis dreifach, gleich oder verschieden substituiertes Phenyl, wobei als Substituenten Fluor, Chlor, Brom, Nitro, Cyano, Methyl, Ethyl, n- oder i-Propyl, n-, i-, s- oder t-Butyl; Methoxy, Ethoxy, n- oder i-Propoxy, n-, i-, s- oder t-Butoxy; Methylthio, CF₃, OCF₃, OCHF₂, SCF₃, SCCl₂F, CH₂Br, CH₂Cl sowie gegebenenfalls einfach bis dreifach, gleich oder verschieden durch Fluor, Chlor, Brom, Nitro, Cyano, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Methylthiomethyl, CF₃, OCF₃, OCHF₂, SCF₃, SCCl₂F, CH₂Br, CH₂Cl, Methoxycarbonyl oder Methylsulfonyl oder Trifluormethylsulfonyl substituiertes Phenoxy in Frage kommen.
- Y: steht besonders bevorzugt -CH₂-, -CH(CH₃)-, -CH₂CH₂- oder -CH=CH-.

Bevorzugte erfindungsgemäße Verbindungen sind Stoffe der Formeln (IA) und (IB): 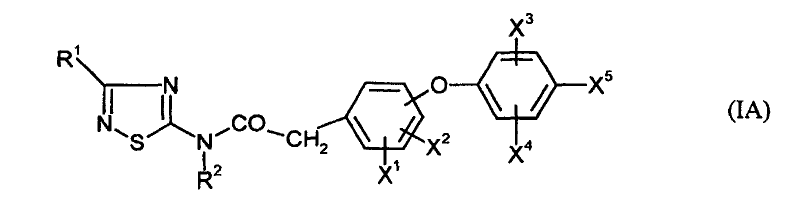 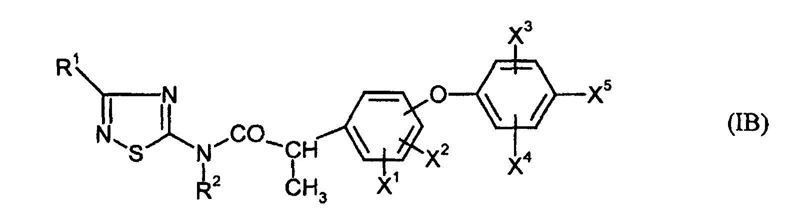 in welchen
- R¹ und R²: für die obengenannten allgemeinen, bevorzugten und besonders bevorzugten Bedeutungen stehen und
- X¹, X², X³, X⁴ und X⁵: für die oben unter R³ allgemein, bevorzugt und besonders bevorzugt für den Phenyl- bzw. Phenoxyrest genannten Substituenten stehen und
- X', X², X³ und X⁴: jeweils auch für Wasserstoff stehen können.

Eine weitere bevorzugte Gruppe von Verbindungen sind solche der Formeln (IA) oder (IB), in welchen der Phenoxyrest in para-Stellung zur NR²-CO-CH₂- bzw. NR²-CO-CH(CH₃)-Gruppe steht, wobei unter diesen Verbindungen jene besonders bevorzugt sind, in welchen die Substituenten X¹, X², X³ und X⁴ für Wasserstoff stehen.

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Im einzelnen seien neben den Herstellungsbeispielen die folgenden Verbindungen der Formel (IC) genannt:

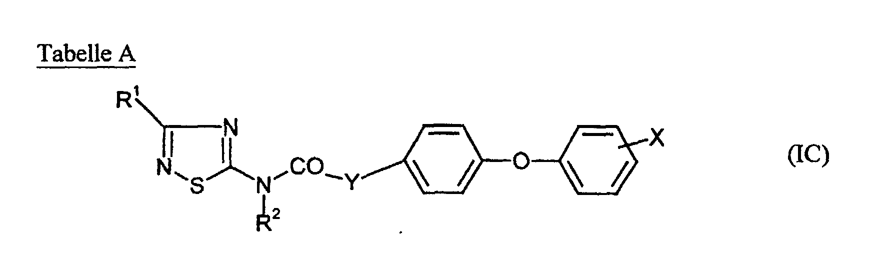

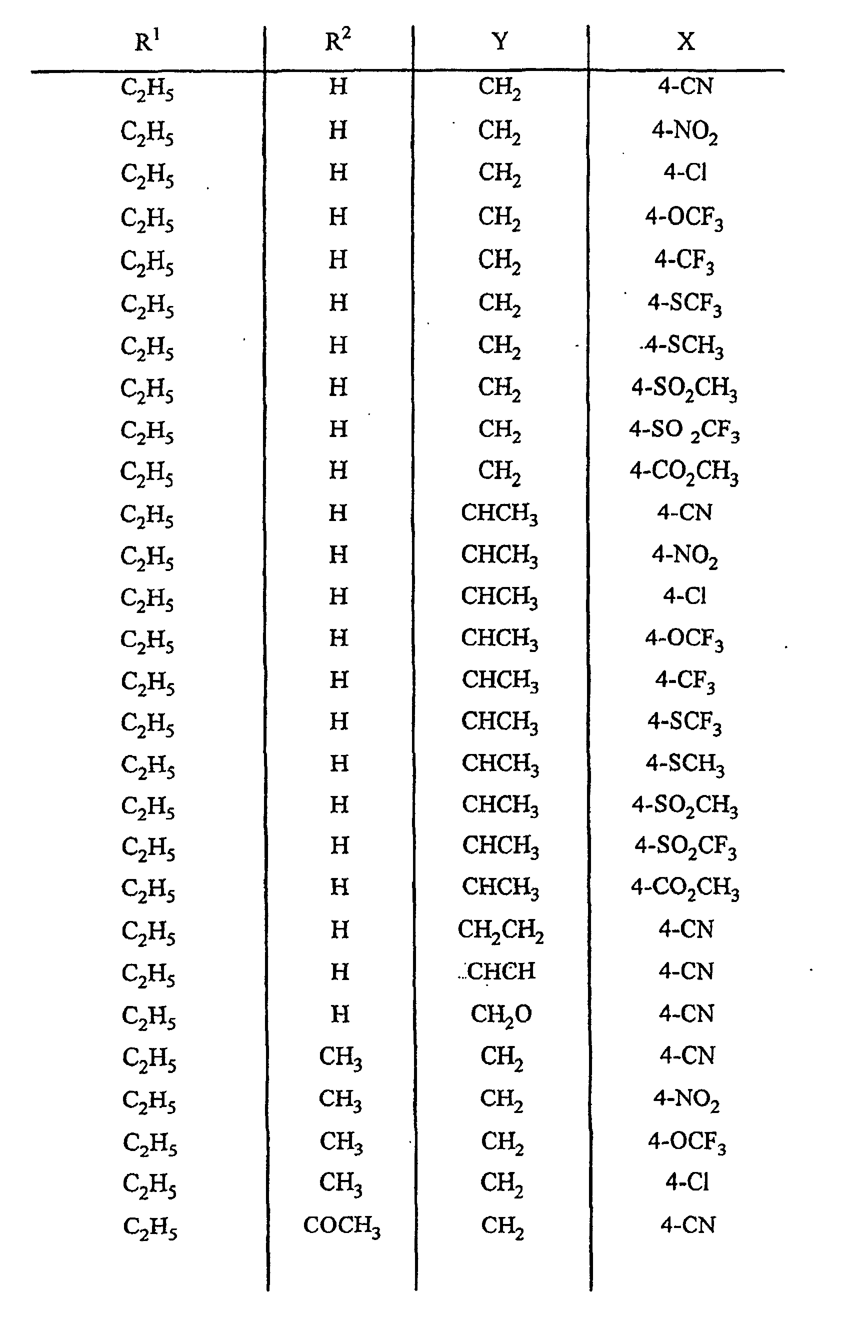

Verwendet man bei der Herstellung von Verbindungen der Formel (I) z.B. 5-Amino-3-methyl-1,2,4-thiadiazol und [4-(4-Cyano)phenoxy]phenyl-essigsäurechlorid als Ausgangsstoffe, so kann der Reaktionsablauf des erfindungsgemäßen Verfahrens durch das folgende Reaktionsschema wiedergegeben werden:

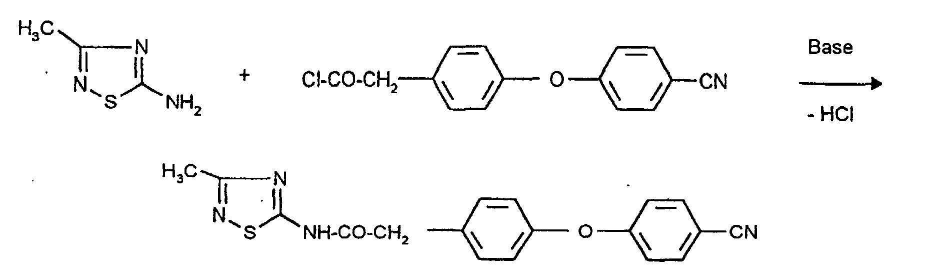

Die beim erfindungsgemäßen Verfahren als Ausgangsstoffe zu verwendenden 5-Amino-1,2,4-thiadiazole der Formel (II) sind bekannt (vgl. z.B. EP-A-0 455 356; Gazz. Chim. Ital. 1977, 107, S. 1ff oder Chem. Ber. 195, 87, S. 57ff) und/oder lassen sich nach bekannten Methoden herstellen (vgl. z.B. die o.g. Literaturstellen).

Die weiterhin beim erfindungsgemäßen Verfahren als Ausgangsstoffe zu verwendenden Säurehalogenide der Formel (III) sind allgemein bekannte Verbindungen der organischen Chemie. In der Formel (III) steht Hal vorzugsweise für Chlor oder Brom.

Das erfindungsgemäße Verfahren zur Herstellung der Verbindungen der Formel (I) wird in Gegenwart eines Verdünnungsmittels durchgeführt. Als Verdünnungsmittel können alle üblichen Lösungsmittel eingesetzt werden.

Vorzugsweise verwendbar sind gegebenenfalls halogenierte aliphatische oder aromatische Kohlenwasserstoffe, Ether oder Nitrile wie z.B. Cyclohexan, Toluol, Chlorbenzol, Choroform, Dichlormethan, Dichlorethan, Dioxan, Tetrahydrofuran, Diethylether oder Acetonitril.

Das erfindungsgemäße Verfahren zur Herstellung der Verbindungen der Formel (I) wird in Gegenwart einer Base durchgeführt.

Als Basen können alle üblichen Protonenakzeptoren eingesetzt werden. Vorzugsweise verwendbar sind Alkali- oder Erdalkalihydroxide, Alkali- oder Erdalkalicarbonate oder -hydrogencarbonate oder Stickstoffbasen. Genannt seien beispielsweise Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktionstemperaturen können bei dem erfindungsgemäßen Verfahren in einem größeren Bereich variiert werden. Im allgemeinen arbeitet man bei Temperaturen zwischen -40°C und +200°C, bevorzugt zwischen 0°C und 100°C.

Bei der Durchführung des erfindungsgemäßen Verfahrens setzt-man im allgemeinen pro Mol 5-Amino-1,2,4-thiadiazol der Formel (II) 1 bis 2 Mol, vorzugsweise 1 bis 1,5 Mol an Säurehalogenid der Formel (III) ein.

Dabei erweist es sich in manchen Fällen als vorteilhaft, die 5-Amino-1,2,4-thiadiazole der Formel (II) in Form ihrer Hydrohalogenide, wie insbesondere als Hydrochloride einzusetzen.

Aufarbeitung und Isolierung der Endprodukte erfolgen in allgemein bekannter Art und Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsund Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören.

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp.; Aphelenchoides spp., Longidorus spp., Xiphinema spp., Trichodorus spp..

Die erfindungsgemäßen Verbindungen der Formel (I) zeichnen sich insbesondere durch eine hohe insektizide und akarizide Wirksamkeit aus.

Sie lassen sich mit besonders gutem Erfolg zur Bekämpfung von pflanzenschädigenden Insekten einsetzen, wie beispielsweise gegen die Meerrettichblattkäfer-Larven (Phaedon cochlaeriae), die Raupen der Kohlschabe (Plutella maculipennis), die grüne Reiszikade (Nephotettix cinctriceps), die Pfirsichblattlaus (Mycus persicae) und die Raupen des Eulenfalters (Spodoptera frugiperda) oder zur Bekämpfung von pflanzenschädigenden Milben, wie beispielsweise gegen die gemeine Spinnmilbe (Tetranychus urticae).

Die erfindungsgemäßen Stoffe weisen eine starke mikrobizide Wirkung auf und können zur Bekämpfung von unerwünschten Mikroorganismen, wie Fungi und Bakteria, im Pflanzenschutz und im Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Psdeudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit gutem Erfolg zur Bekämpfung von Krankheiten im Wein-, Obst- und Gemüseanbau, wie beispielsweise gegen Plasmopara-Arten, einsetzen. Mit gutem Erfolg werden auch Getreidekrankheiten, wie beispielsweise Cochliobolus-, Leptosphaeria- und Erysiphe-Arten, oder Reiskrankheiten, wie beispielsweise Pyricularia-Arten, bekämpft. Ferner lassen sich die erfindungsgemäßen Verbindungen auch zur Steigerung des Ernteertrags von Kulturpflanzen einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe bzw. Mittel gegen Pilze, insbesondere Schimmelpilze, sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole und Feinstverkapselungen in polymeren Stoffen und in Hüllmassen für Saatgut, sowie ULV-Kalt und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck- gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticdiin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfosufen(IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Boerdeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozcen(PCNB),
Schwefel und Schwefel-Zubereitungen,
Tebuconazol, Tecloftalam, Tecnhazen, Tetcyclacis, Tetraconazol, Thiabendazol, Thicyofen, Thifluzamide, Tthiophanate-methyl, Thiram, Tioxymid, Tolclofosmethyl, Tolylfluanid, Triadimefon, Triadimenol, Triazbutanil, Triazoxid,
Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)sulfonyl]-5-(trichlormethyl)-1,3,4-thiadiazol,
2-[[6-Deoxy-.4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan-[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-onm,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-vitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-.oxo-3 -oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin -Mono-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin, Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die Wirkstoffe können als solche, in Form ihrer handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw.. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise. von 0,05 bis 1,0 Gew.-% bezogen auf das zu schützende Material.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie

Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz,- Phenolharz, Kohlenwasserstoffharz wie Inden--Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, genannt.

Die Herstellung und die Verwendung der erfindungsgemäßen Wirkstoffe gehen aus den nachfolgenden Beispielen hervor.

### Herstellungsbeispiele

### Beispiel 1

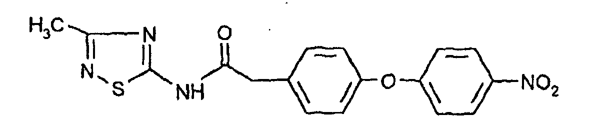

Zu einer Lösung von 2,00g (0,0174 mol) 5-Amino-3-Methyl-1,2,4-thiadiazol und 1,50 g (0,0209 mol) Pyridin in 80 ml Acetonitril wird eine Lösung von 6,10 g (0,0209 mol) [4-(4-Nitro)phenoxy]-phenylessigsäurechlorid in 20 ml Acetonitril getropft. Man rührt 18 Stunden bei 25°C und engt anschließend bis zur Trockne ein. Das Reaktionsgemisch wird in Wasser/Ethylacetat aufgenommen und die organische Phase mehrmals mit 10%-iger Natronlauge gewaschen.

Nach Trocknen und Einengen erhält man 3,2 g (44 % der Theorie) 3-Methyl-5-[4-(4-nitro)phenoxy]phenylacetyl-amino-1,2,4-thiadiazol vom Schmelzpunkt 168-170°C.

Analog bzw. gemäß den allgemeinen Angaben zur Herstellung erhält man die folgenden Verbindungen der Formel (I):

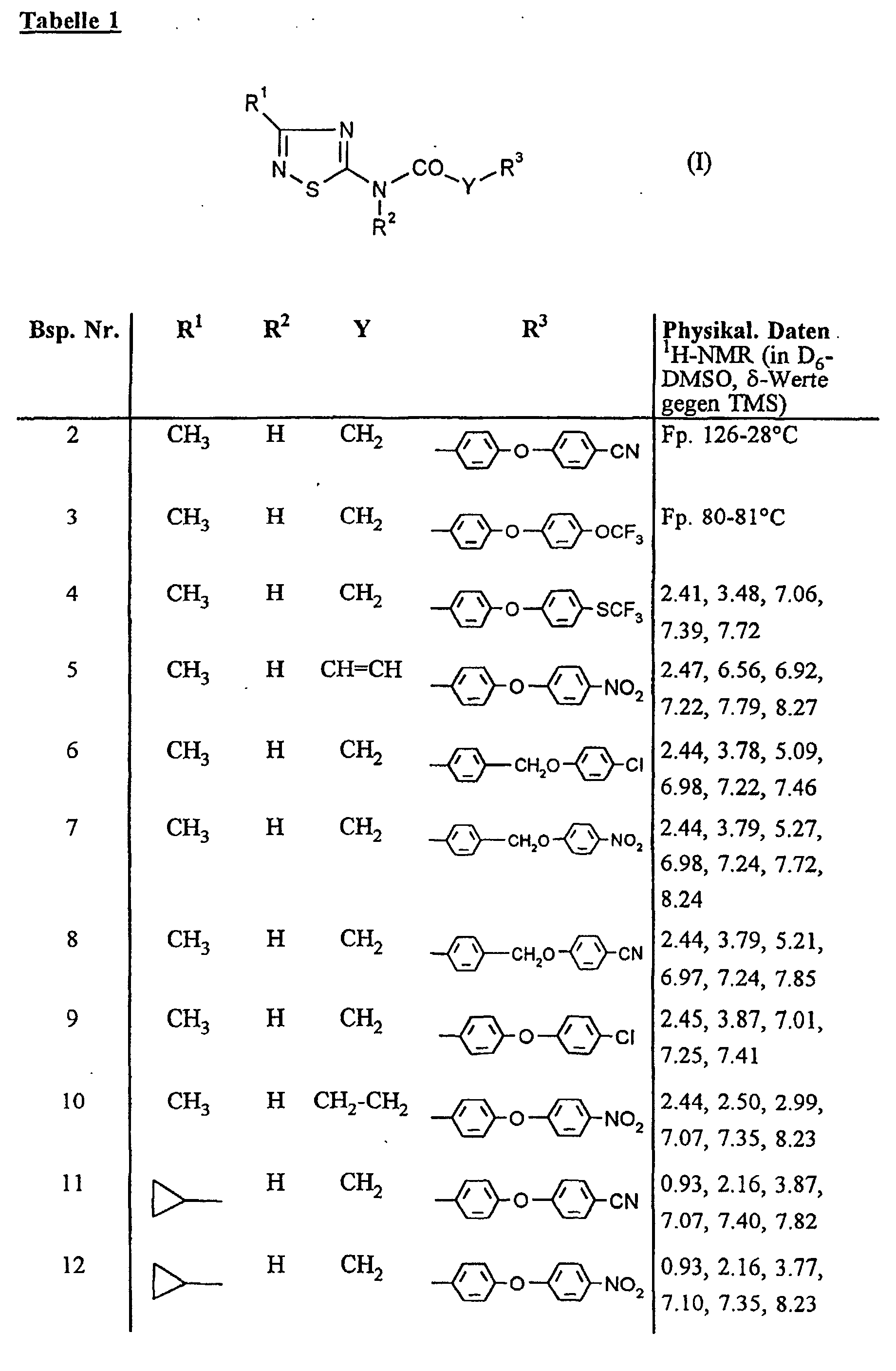

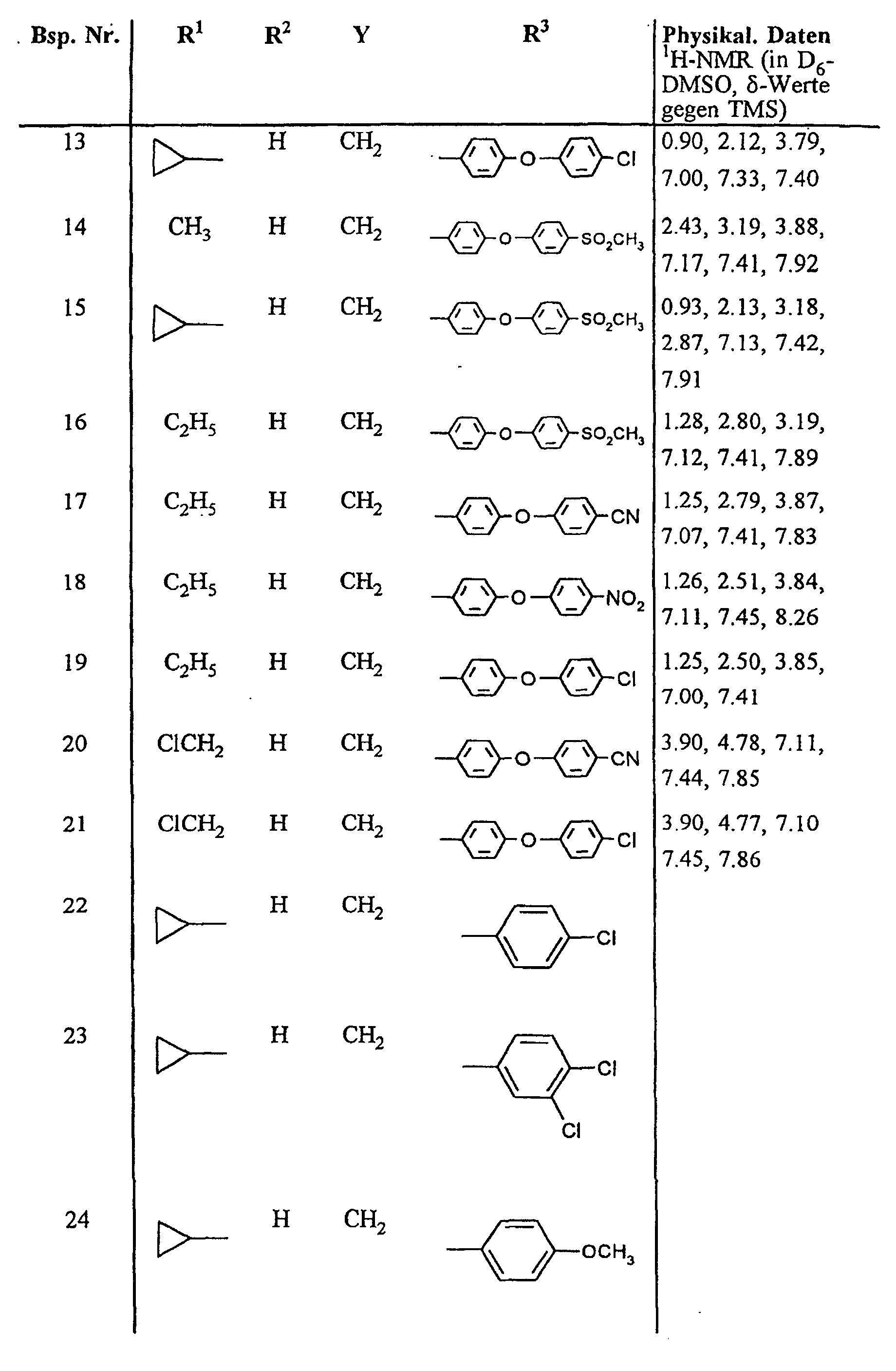

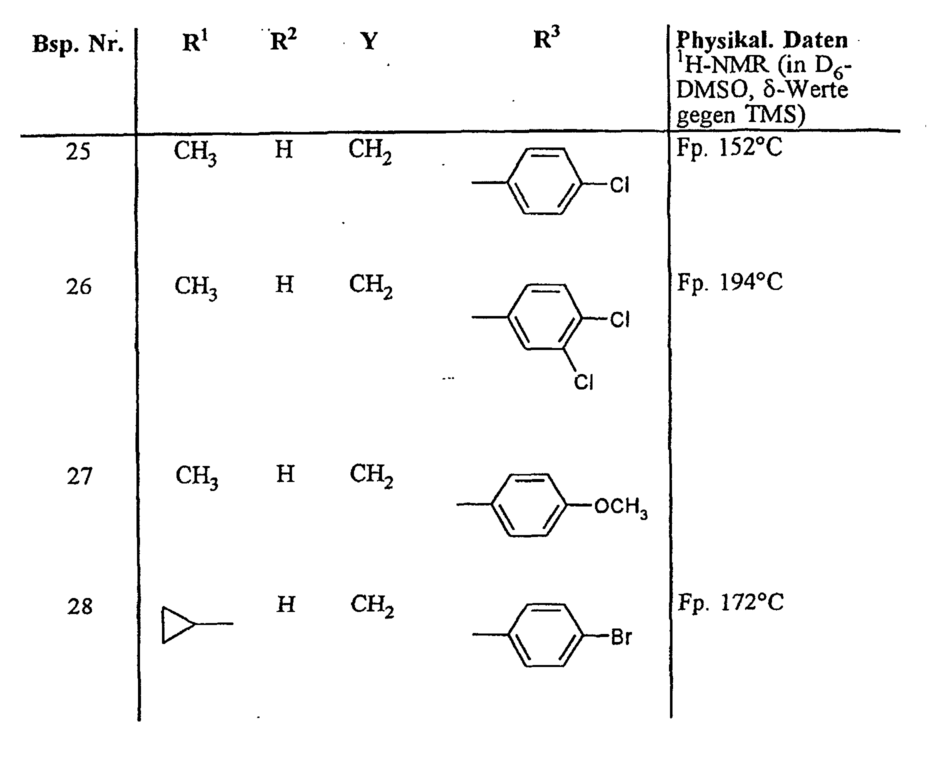

### Anwendungsbeispiele

### Beispiel A

### Phaedon-Larven-Test

| | | |
|---|---|---|
| Lösungsmittel | 7 Gewichtsteile | Dimethylfomamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen 1, 2 und 3 bei einer beispielhaften Wirkstoffkonzentration von 0,1 % eine Abtötung von 100 % nach 7 Tagen.

### Beispiel B

### Plutella-Test

| | | |
|---|---|---|
| Lösungsmittel | 7 Gewichtsteile | Dimethylfomamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen 1, 2 und 3 bei einer beispielhaften Wirkstoffkonzentration von 0,1 % eine Abtötung von 100 % nach 7 Tagen.

### Beispiel C

### Spodoptera Frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen 1, 2 und 3 bei einer beispielhaften Wirkstoffkonzentration von 0,1 % eine Abtötung von 100 % nach 7 Tagen.

### Beispiel D

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen 1, 2 bzw. 3 bei einer beispielhaften Wirkstoffkonzentration von 0,1 % eine Abtötung von 100 % nach 6 Tagen.

### Beispiel E

### Myzus-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt -das Konzentrat mit Wasser auf die gewünschte Konzentration.

Keimlinge der Dicken Bohne (Vicia faba), die von der Grünen Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen 1, 2 und 3 bei einer beispielhaften Wirkstoffkonzentration von 0,1 % eine Abtötung von mindestens 90 % nach 6 Tagen.

### Beispiel F

### Tetranychus-Test (OP-resistent/Tauchbehandlung)

| | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkte z.B. die Verbindung gemäß dem Herstellungsbeispiel 3 bei einer beispielhaften Wirkstoffkonzentration von 0,1 % eine Abtötung von 100 % nach 13 Tagen.

### Beispiel G

### Test mit Boophilus microplus resistent / SP resistenter Parkhurst-Stamm

| | |
|---|---|
| Testtiere | adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test hatte z.B. die Verbindung gemäß Herstellungsbeispiel 2 bei einer beispielhaften Wirkstoffkonzentration von 20 µg/Tier eine Wirkung von 100 %.

### Beispiel H

### Test mit Fliegenlarven / Entwicklungshemmende Wirkung

| | |
|---|---|
| Testtiere | Alle larvalen Stadien von Lucilia cuprina (OP-resistent) [Puppen und Adulte (ohne Kontakt zum Wirkstoff)] |
| Lösungsmittel | 35 Gewichtsteile Ethylenglykolmonomethylether 35 Gewichtsteile Nonylphenolpolyglykolether |

Zwecks Herstellung einer geeigneten Formulierung vermischt man 3 Gewichtsteile Wirkstoff mit 7 Teilen des oben angegebenen Lösungsmittel-Emulgator-Gemisches und verdünnt das so erhaltene Emulsionskonzentrat mit Wasser auf die jeweils gewünschte Konzentration.

30 bis 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % rel. Feuchte ±10 %) aufbewahrt. Die Wirkungskontrolle erfolgt nach 24 Stunden und 48 Stunden (larvizide Wirkung). Nach dem Auswandern der Larven (ca. 72 h) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 h (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Fliegenentwicklung, bzw. ein Entwicklungsstillstand vor dem Adulten-Stadium. Dabei bedeutet 100 % larvizide Wirkung, daß nach 48 Stunden alle Larven abgestoben sind. 100 % entwicklungsinhibitorische Wirkung bedeutet, daß keine adulte Fliegen geschlüpft sind.

In diesem Test hatten z.B. die Verbindungen gemäß den Herstellungsbeispielen 2 und 3 bei einer beispielhaften Wirkstoffkonzentration von 1000 ppm eine Wirkung von 100 %.

### Beispiel I

### Pyricularia-Test (Reis) / protektiv

| | |
|---|---|
| Lösungsmittel | 2,5 Gewichtsanteile Aceton |
| Emulgator | 0,06 Gewichtsanteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit werden junge Reispflanzen mit der Wirkstoffzubereitung taufeucht bespritzt. 1 Tag nach dem Abtrocknen des Spitzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

In diesem Test hatten z.B. die Verbindungen gemäß den Herstellungsbeispielen 3, 4, 22 und 23 bei einer beispielhaften Wirkstoffaufwandmenge von 750 g/ha und einer Einwirkzeit von einem Tag einen Wirkungsgrad von mindestens 70%, bezogen auf die unbehandelte Kontrolle.

Ebenso die Verbindungen gemäß den Herstellungsbeispielen 1, 2, 3 und 26 bei einer Einwirkzeit von 4 Tagen.

### Beispiel J

### Plasmopara-Test (Rebe) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100% relativer Luftfeuchtigkeit. Anschließend werden die Pflanzen 5 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet ein Wirkungsgrad von 0%, daß der Befall auf den behandelten Pflanzen demjenigen der unbehandelten Kontrolle entspricht, während ein Wirkungsgrad von 100% bedeutet, daß auf den behandelten Pflanzen kein Befall beobachtet wird.

In diesem Test hatte z.B. die Verbindung gemäß Herstellungsbeispiel 2 bei einer beispielhaften Wirkstoffaufwandmenge von 100 g/h einen Wirkungsgrad von 100%.

### Beispiel K

### Sphaerotheca-Test (Gurke) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages weden die Pflanzen mit einer wäßrigen Sporensuspension von Sphaerotheca fuliginea inokuliert. Die Pflanzen werden bei ca. 23°C und einer relativen Luftfeuchtigkeit von ca. 70% im Gewächshaus aufgestellt.

10 Tage nach der Inokulation erfolgt die Auswertung. Dabei bedeutet ein Wirkungsgrad von 0%, daß der Befehl auf den behandelten Pflanzen demjenigen der unbehandelten Kontrolle entspricht, während ein Wirkungsgrad von 100% bedeutet, daß auf den behandelten Pflanzen kein Befall beobachtet wird.

In diesem Test hatte z.B. die Verbindung gemäß Herstellungsbeispiel 2 bei einer beispielhaften Wirkstoffaufwandmenge von 100 g/ha einen Wirkungsgrad von 98%.

### Beispiel L

### Uncinula - Test (Rebe) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstsoffzubereitung taufeucht besprüht. Nach Antrocknen des Spritzbelages werden die Pflanzen mit einer wäßrigen Sporensuspension von **Uncinula necator** inokuliert. Die Pflanzen werden im Gewächshaus bei ca. 23°C und einer relativen Luftfeuchtigkeit von ca. 70% aufgestellt.

14 Tage nach der Inokulation erfolgt die Auswertung. Dabei bedeutet ein Wirkungsgrad von 0%, daß der Befall auf den behandelten Pflanzen demjenigen der unbehandelten Kontrolle entspricht, während ein Wirkungsgrad von 100% bedeutet, daß auf den behandelten Pflanzen kein Befall beobachtet wird.

In diesem Test hatte z.B. die Verbindung gemäß Herstellungsbeispiel 2 bei einer beispielhaften Wirkstoffaufwandmenge von 100 g/ha einen Wirkungsgrad von 100%.

### Beispiel M

### Venturia - Test (Apfel) / protektiv

| | |
|---|---|
| Lösungsmittel | 47 Gewichtsteile Aceton |
| Emulgator | 3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100% relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

12 Tage nach der Inokulation erfolgt die Auswertung. Dabei bedeutet ein Wirkungsgrad von 0%, daß der Befall auf den behandelten Pflanzen demjenigen der unbehandelten Kontrolle entspricht, während ein Wirkungsgrad von 100% bedeutet, daß auf den behandelten Pflanzen kein Befall beobachtet wird.

In diesem Test hatte z.B. die Verbindung gemäß Herstellungsbeispiel 2 bei einer beispielhaften Wirkstoffaufwandmenge von 100 g/ha einen Wirkungsgrad von 94%.

## Patentansprüche

1. Verbindungen der Formel (I) 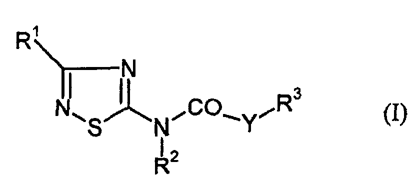 in welcher
R¹ für C₁-C₄-Alkyl, C₁-C₄-Halogenalkyl mit 1 bis 5 gleichen oder verschiedenen Halogenatomen, wie Fluor-, Chlor- und Bromatomen, C₁-C₄-Alkoxy-C₁-C₄-alkyl, C₁-C₄-Alkylthio-C₁-C₄-alkyl, C₁-C₄-Alkoxy, C₁-C₄-Alkylthio oder für gegebenenfalls einfach bis dreifach, gleich oder verschieden durch C₁-C₄-Alkyl oder Halogen substituiertes C₃-C₆-Cycloalkyl steht,
R² für Wasserstoff, C₁-C₄-Alkyl, C₁-C₄-Halogenalkyl mit 1 bis 5 gleichen oder verschiedenen Halogenatomen, wie Fluor- und Chloratomen, C₁-C₄-Alkoxy-C₁-C₄-alkyl, C₁-C₄-Alkyl-carbonyl, C₁-C₄-Alkylsulfonyl, jeweils gegebenenfalls einfach bis dreifach, gleich oder verschieden im Phenylring substituiertes Phenylcarbonyl, Phenylsulfonyl oder Benzyl steht, wobei als Substituenten jeweils Halogen, Nitro, Cyano, C₁-C₄-Alkyl, C₁-C₂-Halogenalkyl mit 1 bis 5 gleichen oder verschiedenen Halogenatomen, wie Fluor- und Chloratome, C₁-C₄-Alkoxy, C₁-C₄-Alkylthio, C₁-C₂-Halogenalkoxy mit 1 bis 5 gleichen oder verschiedenen Halogenatomen, wie Fluor- und Chloratome oder C₁-C₂-Halogenalkylthio mit 1 bis 5 gleichen oder verschiedenen Halogenatomen, wie Fluor- und Chloratome in Frage kommen, oder fiir gegebenenfalls einfach bis dreifach, gleich oder verschieden durch C₁-C₄-Alkyl oder Halogen substituiertes C₃-C₆-Cycloalkyl steht,
R³ für gegebenenfalls einfach bis dreifach, gleich oder verschieden substituiertes Phenyl steht, wobei als Substituenten Halogen, Nitro, Cyano, C₁-C₁₂-Alkyl, C₁-C₁₂-Halogenalkylmit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₆-Cycloalkyl sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratome oder C₁-C₄-Alkoxy-carbonyl substituiertes Phenyl, Phenoxy, Phenylthio oder Benzyl in Frage kommen,
oder für jeweils gegebenenfalls einfach bis dreifach, gleich oder verschieden substituiertes C₃-C₈-Cycloalkyl oder C₅-C₈-Cycloalkenyl steht, wobei als Substituenten C₁-C₄-Alkyl, C₁-C₄-Halogenalkyl mit 1 bis 5 gleichen oder verschiedenen Halogenatomen, wie Fluorund Chloratome, C₁-C₄-Alkoxy, C₁-C₄-Halogenalkoxy mit 1 bis 5 gleichen oder verschiedenen Halogenatomen, wie Fluor- und Chloratome, C₃-C₈-Cycloalkyl, sowie gegebenenfalls einfach bis dreifach, gleich oder verschieden durch Halogen, Cyano, Nitro, C₁-C₄-Alkyl, C₁-C₄-Halogenalkyl mit 1 bis 5 gleichen oder verschiedenen Halogenatomen, wie Fluor- und Choratome, C₁-C₄-Alkoxy oder C₁-C₄-Halogenalkoxy mit 1 bis 5 gleichen oder verschiedenen Halogenatomen, wie Fluor- und Chloratome, substituiertes Phenyl in Frage kommen und
Y für C₁-C₆-Alkylen, C₁-C₆-Hydroxyalkylen, C₁-C₄-Alkoxy-C₁-C₆-alkylen, C₁-C₄-Alkylcarbonyloxy-C₁-C₆-alkylen, Cyano-C₁-C₆-alkylen, C₁-C₄-Halogenalkylen mit 1 bis 5 gleichen oder verschiedenen Halogenatomen, wie Fluor- und Chloratome; gegebenenfalls einfach bis dreifach, gleich oder verschieden durch Fluor, Chlor oder Methyl substituiertes C₃-C₆-Cycloalkyl-C₁-C₄-alkylen, C₂-C₄-Alkenylen oder C₁-C₄-Alkylenoxy steht.

2. Verbindungen der Formel (I) gemäß Anspruch 1, in welcher
R¹ für Methyl, Ethyl, n- oder i-Propyl, n-, i-, s- oder t-Butyl; CH₂F, CHF₂, CF₃, CH₂Cl, CH₂Br, CHClCH₃; Methoxy, Ethoxy, Methoxymethyl, Ethoxymethyl; Methylthiomethyl oder Cyclopropyl steht,
R² für Wasserstoff, Methyl, Ethyl, n- oder i-Propyl; Methoxymethyl, Ethoxymethyl, n-Propoxymethyl, n-Butoxymethyl; Methylcarbonyl, Methylsulfonyl; jeweils gegebenenfalls einfach oder zweifach, gleich oder verschieden durch Fluor, Chlor, Methyl oder Trifluormethyl substituiertes Phenylcarbonyl oder Benzyl; oder Cyclopropyl steht,
R³ xfür gegebenenfalls einfach bis dreifach, gleich oder verschieden substituiertes Phenyl steht, wobei als Substituenten Halogen, Nitro, Cyano , C₁-C₁₂-Alkyl, C₁-C₁₂-Halogenalkylmit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- und Chloratomen, C₂-C₄-Alkenyl, C₂-C₄-Halogenalkenyl mit 1 bis 5 gleichen oder verschiedenen Halogenatomen, wie Fluor- und Chloratomen, sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- oder Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratome oder C₁-C₄-Alkoxy-carbonyl substituiertes Phenoxy oder Phenylthio in Frage kommen und
Y für eine der Gruppen
-CH₂-, -CH(CH₃)-, -CH(C₂H₅)-, -CH(n-C₃H₇)-, -CH(i-C₃H₇)-, -CH₂CH₂-, -CH(OH)-, -CH(OCH₃)-, -CH(O-CO-CCH₃)-, -CH(CN)-, -CHF-, -CHCl-, -CH-[ 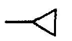 ]-, -CH=CH- oder -CH₂O- steht.

3. Verbindungen der Formel (I) gemäß Anspruch 1, in welcher
R¹ für Methyl, Ethyl, n- oder i-Propyl, n-, i-, s- oder t-Butyl; CH₂F, CHF₂, CF₃, CH₂Cl, CH₂Br, Methoxy, Ethoxy oder Cyclopropyl steht,
R² für Wasserstoff, Methyl, Ethyl, Methoxymethyl, Ethoxymethyl, Methylcarbonyl, Phenylcarbonyl oder Methylsulfonyl steht,
R³ für gegebenenfalls einfach bis dreifach, gleich oder verschieden substituiertes Phenyl steht, wobei als Substituenten Fluor, Chlor, Brom, Nitro, Cyano, Methyl, Ethyl, n- oder i-Propyl, n-, i-, s- oder t-Butyl; Methoxy, Ethoxy, n- oder i-Propoxy, n-, i-, s- oder t-Butoxy; Methylthio, CF₃, OCF₃, OCHF₂, SCF₃, SCCl₂F, CH₂Br, CH₂Cl sowie gegebenenfalls einfach bis dreifach, gleich oder verschieden durch Fluor, Chlor, Brom, Nitro, Cyano, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Methylthiomethyl, CF₃, OCF₃, OCHF₂, SCF₃, SCCl₂F, CH₂Br, CH₂Cl, Methoxycarbonyl oder Methylsulfonyl oder Trifluormethylsulfonyl substituiertes Phenoxy in Frage kommen und
Y -CH₂-, -CH(CH₃)-, -CH₂CH₂- oder -CH=CH- steht.

4. Verfahren zur Herstellung von Verbindungen der Formel (I) gemäß Anspruch 1, **dadurch gekennzeichnet, dass** man
5-Amino-1,2,4-thiadiazole der Formel (II) 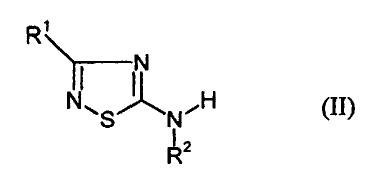 in welcher
R¹ und R² die in Anspruch 1 angegebene Bedeutung haben,
mit Säurehalogeniden der Formel (III)
Hal―CO―Y―R³ (III)
in welcher
R³ und Y die in Anspruch 1 angegebene Bedeutung haben und
Hal für Halogen steht,
in Gegenwart einer Base und in Gegenwart eines Verdünnungsmittels umsetzt.

5. Schädlingsbekämpfungsmittel, **gekennzeichnet durch** einen Gehalt an mindestens einer Verbindung der Formel (I) gemäß Anspruch 1.

6. Verwendung von Verbindungen der Formel (I) gemäß Anspruch 1 zur Bekämpfung von Schädlingen.

7. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

8. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

9. Verwendung von Verbindungen der Formel (I) gemäß Anspruch 1 zur Herstellung von Schädlingsbekämpfungsmitteln.

## Claims

1. Compounds of the formula (I) 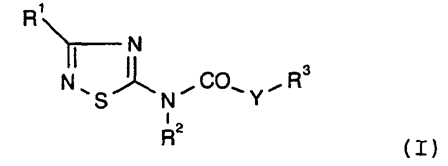 in which
R¹ represents C₁-C₄-alkyl, C₁-C₄-halogenoalkyl having 1 to 5 identical or different halogen atoms, such as fluorine, chlorine and bromine atoms, C₁-C₄-alkoxy-C₁-C₄-alkyl, C₁-C₄-alkyl-thio-C₁-C₄-alkyl, C₁-C₄-alkoxy, C₁-C₄-alkylthio or C₃-C₆-cycloalkyl which is optionally mono- to trisubstituted by identical or different C₁-C₄-alkyl or halogen substituents,
R² represents hydrogen, C₁-C₄-alkyl, C₁-C₄-halogenoalkyl having 1 to 5 identical or different halogen atoms, such as fluorine and chlorine atoms, C₁-C₄-alkoxy-C₁-C₄-alkyl, C₁-C₄-alkyl-carbonyl, C₁-C₄-alkylsulphonyl, phenylcarbonyl, phenylsulphonyl or benzyl, each of which is optionally mono- to trisubstituted on the phenyl ring by identical or different substituents from the group consisting of halogen, nitro, cyano, C₁-C₄-alkyl, C₁-C₂-halogenoalkyl having 1 to 5 identical or different halogen atoms, such as fluorine and chlorine atoms, C₁-C₄-alkoxy, C₁-C₄-alkylthio, C₁-C₂-halogenoalkoxy having 1 to 5 identical or different halogen atoms, such as fluorine and chlorine atoms, or C₁-C₂-halogenoalkylthio having 1 to 5 identical or different halogen atoms, such as fluorine and chlorine atoms, or represents C₃-C₆-cycloalkyl which is optionally mono- to trisubstituted by identical or different C₁-C₄-alkyl or halogen substituents,
R³ represents phenyl which is optionally mono- to trisubstituted by identical or different substituents from the group consisting of halogen, nitro, cyano, C₁-C₁₂-alkyl, C₁-C₁₂-halogenoalkyl having 1 to 5 identical or different halogen atoms, such as fluorine and chlorine atoms, C₁-C₁₂-alkoxy, C₁-C₁₂-halogenoalkoxy having 1 to 5 identical or different halogen atoms, such as fluorine and chlorine atoms, C₁-C₁₂-alkylthio, C₁-C₁₂-halogenoalkylthio having 1 to 5 identical or different halogen atoms, such as fluorine and chlorine atoms, C₂-C₁₂-alkenyl, C₂-C₁₂-halogenoalkenyl having 1 to 5 identical or different halogen atoms, such as fluorine and chlorine atoms, C₃-C₆-cycloalkyl which is optionally mono- to trisubstituted by identical or different C₁-C₄-alkyl or halogen substituents, and phenyl, phenoxy, phenylthio or benzyl, each of which is optionally mono- to trisubstituted by identical or different substituents from the group consisting of halogen, nitro, cyano, C₁-C₄-alkyl, C₁-C₄-halogenoalkyl having 1 to 5 identical or different halogen atoms, such as fluorine and chlorine atoms, C₁-C₄-alkoxy, C₁-C₄-halogenoalkoxy having 1 to 5 identical or different halogen atoms, such as fluorine and chlorine atoms, C₁-C₄-alkylthio, C₁-C₄-halogenoalkylthio having 1 to 5 identical or different halogen atoms, such as fluorine and chlorine atoms, C₁-C₄-alkylthio-C₁-C₄-alkyl, C₁-C₄-alkylsulphonyl, C₁-C₄-halogenoalkylsulphonyl having 1 to 5 identical or different halogen atoms, such as fluorine and chlorine atoms, and C₁-C₄-alkoxycarbonyl,
or represents C₃-C₈-cycloalkyl or C₅-C₈-cycloalkenyl, each of which is optionally mono- to trisubstituted by identical or different substituents from the group consisting of C₁-C₄-alkyl, C₁-C₄-halogenoalkyl having 1 to 5 identical or different halogen atoms, such as fluorine and chlorine atoms, C₁-C₄-alkoxy, C₁-C₄-halogenoalkoxy having 1 to 5 identical or different halogen atoms, such as fluorine and chlorine atoms, C₃-C₈-cycloalkyl, and phenyl which is optionally mono- to trisubstituted by identical or different substituents from the group consisting of halogen, cyano, nitro, C₁-C₄-alkyl, C₁-C₄-halogenoalkyl having 1 to 5 identical or different halogen atoms, such as fluorine and chlorine atoms, C₁-C₄-alkoxy and C₁-C₄-halogenoalkoxy having 1 to 5 identical or different halogen atoms, such as fluorine and chlorine atoms, and
Y represents C₁-C₆-alkylene, C₁-C₆-hydroxy-alkylene, C₁-C₄-alkoxy-C₁-C₆-alkylene, C₁-C₄-alkylcarbonyloxy-C₁-C₆-alkylene, cyano-C₁-C₆-alkylene, C₁-C₄-halogenoalkylene having 1 to 5 identical or different halogen atoms, such as fluorine and chlorine atoms; or C₃-C₆-cyclo-alkyl-C₁-C₄-alkylene, C₂-C₄-alkenylene or C₁-C₄-alkyleneoxy, each of which is optionally mono- to trisubstituted by identical or different substituents from the group consisting of fluorine, chlorine and methyl.

2. Compounds of the formula (I) according to Claim 1 in which
R¹ represents methyl, ethyl, n- or i-propyl, n-, i-, s- or t-butyl; CH₂F, CHF₂, CF₃, CH₂Cl, CH₂Br, CHClCH₃; methoxy, ethoxy, methoxymethyl, ethoxymethyl; methylthiomethyl or cyclopropyl,
R² represents hydrogen, methyl, ethyl, n- or i-propyl; methoxymethyl, ethoxymethyl, n-propoxymethyl, n-butoxymethyl; methylcarbonyl, methylsulphonyl; phenylcarbonyl or benzyl, each of which is optionally mono- or disubstituted by identical or different substituents from the group consisting of fluorine, chlorine, methyl and trifluoromethyl; or cyclopropyl,
R³ represents phenyl which is optionally mono- to trisubstituted by identical or different substituents from the group consisting of halogen, nitro, cyano, C₁-C₁₂-alkyl, C₁-C₁₂-halogenoalkyl having 1 to 5 identical or different halogen atoms, such as fluorine and chlorine atoms, C₁-C₄-alkoxy, C₁-C₄-halogenoalkoxy having 1 to 5 identical or different halogen atoms, such as fluorine and chlorine atoms C₁-C₄-alkylthio C₁-C₄-halogenoalkylthio having 1 to 5 identical or different halogen atoms, such as fluorine and chlorine atoms, C₂-C₄-alkenyl, C₂-C₄-halogenoalkenyl having 1 to 5 identical or different halogen atoms, such as fluorine and chlorine atoms, and phenoxy or phenylthio, each of which is optionally mono- to trisubstituted by identical or different substituents from the group consisting of halogen, nitro, cyano, C₁-C₄-alkyl, C₁-C₄-halogenoalkyl having 1 to 5 identical or different halogen atoms, such as fluorine and chlorine atoms, C₁-C₄-alkoxy, C₁-C₄-halogenoalkoxy having 1 to 5 identical or different halogen atoms, such as fluorine and chlorine atoms, C₁-C₄-alkylthio, C₁-C₄-halogenoalkylthio having 1 to 5 identical or different halogen atoms, such as fluorine and chlorine atoms, C₁-C₄-alkylthio-C₁-C₄-alkyl, C₁-C₄-alkylsulphonyl, C₁-C₄-halogenoalkylsulphonyl having 1 to 5 identical or different halogen atoms, such as fluorine and chlorine atoms, and C₁-C₄-alkoxy-carbonyl, and
Y represents one of the groups
-CH₂-, -CH(CH₃)-, -CH(C₂H₅)-, -CH(n-C₃H₇)-, -CH(i-C₃H₇)-, -CH₂CH₂-, -CH(OH)-, -CH(OCH₃)-, -CH(O-CO-CCH₃)-, -CH(CN)-, -CHF-, -CHCl-, -CH-[ 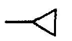 ]-, -CH=CH- or -CH₂O-.

3. Compounds of the formula (I) according to Claim 1 in which
R¹ represents methyl, ethyl, n- or i-propyl, n-, i-, s- or t-butyl; CH₂F, CHF₂, CF₃, CH₂Cl, CH₂Br, methoxy, ethoxy or cyclopropyl,
R² represents hydrogen, methyl, ethyl, methoxymethyl, ethoxymethyl, methylcarbonyl, phenylcarbonyl or methylsulphonyl,
R³ represents phenyl which is optionally mono- to trisubstituted by identical or different substituents from the group consisting of fluorine, chlorine, bromine, nitro, cyano, methyl, ethyl, n- or i-propyl, n-, i-, s- or t-butyl; methoxy, ethoxy, n- or i-propoxy, n-, i-, s- or t-butoxy; methylthio, CF₃, OCF₃, OCHF₂, SCF₃, SCCl₂F, CH₂Br, CH₂Cl, and phenoxy which is optionally mono- to trisubstituted by identical or different substituents from the group consisting of fluorine, chlorine, bromine, nitro, cyano, methyl, ethyl, n- or i-propyl, n-, i-, s- or t-butyl, methoxy, ethoxy, n- or i-propoxy, n-, i-, s- or t-butoxy, methylthio, methylthiomethyl, CF₃, OCF₃, OCHF₂, SCF₃, SCCl₂F, CH₂Br, CH₂Cl, methoxycarbonyl, methylsulphonyl and trifluoromethylsulphonyl, and
Y represents -CH₂-, -CH(CH₃)-, -CH₂CH₂- or -CH=CH-.

4. Process for preparing compounds of the formula (I) according to Claim 1, **characterized in that**
5-amino-1,2,4-thiadiazoles of the formula (II) 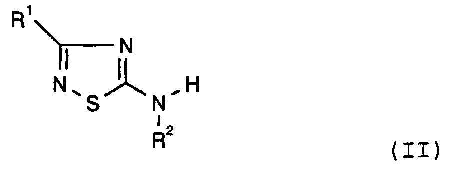 in which
R¹ and R² each have the meanings stated in Claim 1 are reacted with acid halides of the formula (III)
Hal-CO-Y-R³ (III)
in which
R³ and Y each have the meanings stated in Claim 1 and
Hal represents halogen
in the presence of a base and in the presence of a diluent.

5. Pesticides, **characterized in that** they comprise at least one compound of the formula (I) according to Claim 1.

6. Use of compounds of the formula (I) according to Claim 1 for controlling pests.

7. Method for controlling pests, **characterized in that** compounds of the formula (I) according to Claim 1 are allowed to act on pests and/or their habitat.

8. Process for preparing pesticides, **characterized in that** compounds of the formula (I) according to Claim 1 are mixed with extenders and/or surface-active agents.

9. Use of compounds of the formula (I) according to Claim 1 for preparing pesticides.

## Revendications

1. Composés de formule (I) 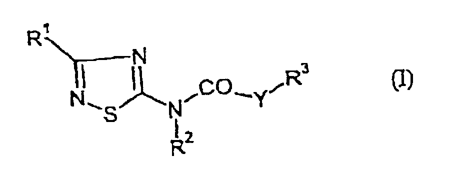 dans laquelle
R¹ est un reste alkyle en C₁ à C₄, halogénalkyle en C₁ à C₄ ayant 1 à 5 atomes d'halogènes identiques ou différents tels que des atomes de fluor, de chlore et de brome, (alkoxy en C₁ à C₄) - (alkyle en C₁ à C₄), (alkylthio en C₁ à C₄)-(alkyle en C₁ à C₄), alkoxy en C₁ à C₄, alkylthio en C₁ à C₄ ou un reste cycloalkyle en C₃ à C₆ et portant éventuellement un à trois substituants, identiques ou différents, alkyle en C₁ à C₄ ou halogéno,
R² représente l'hydrogène, un reste alkyle en C₁ à C₄, halogénalkyle en C₁ à C₄ ayant 1 à 5 atomes d'halogènes identiques ou différents tels que des atomes de fluor et de chlore, un reste (alkoxy en C₁ à C₄)-(alkyle en C₁ à C₄), (alkyle en C₁ à C₄)-carbonyle, alkylsulfonyle en C₁ à C₄, un reste phénylcarbonyle, phénylsulfonyle ou benzyle dont chacun porte éventuellement un à trois substituants identiques ou différents dans le noyau phényle, les substituants considérés dans chaque cas étant des substituants halogéno, nitro, cyano, alkyle C₁ à C₄, halogénalkyle en C₁ ou C₂ ayant 1 à 5 atomes d'halogènes identiques ou différents tels que des atomes de fluor et de chlore, alkoxy en C₁ à C₄, alkylthio en C₁ à C₄, halogénalkoxy en C₁ ou C₂ ayant 1 à 5 atomes d'halogènes identiques ou différents tels que des atomes de fluor et de chlore, ou halogénalkylthio en C₁ ou C₂ ayant 1 à 5 atomes d'halogènes identiques ou différents tels que des atomes de fluor et de chlore, ou un reste cycloalkyle en C₃ à C₆ portant éventuellement un à trois substituants, identiques ou différents, alkyle en C₁ à C₄ ou halogéno,
R³ eest un reste phényle portant éventuellement un à trois substituants identiques ou différents, les substituants considérés étant des substituants halogéno, nitro, cyano, alkyle en C₁ à C₁₂, halogénalkyle en C₁ à C₁₂ ayant 1 à 5 atomes d'halogènes identiques ou différents tels que des atomes de fluor et de chlore, alkoxy en C₁ à C₁₂, halogénalkoxy en C₁ à C₁₂ ayant 1 à 5 atomes d'halogènes identiques ou différents tels que des atomes de fluor et de chlore, alkylthio en C₁ à C₁₂, halogénalkylthio en C₁ à C₁₂ ayant 1 à 5 atomes d'halogènes identiques ou différents tels que des atomes de fluor et de chlore, alcényle en C₂ à C₁₂, halogénalcényle en C₂ à C₁₂ ayant 1 à 5 atomes d'halogènes identiques ou différents tels que des atomes de fluor et de chlore, cycloalkyle en C₃ à C₅ portant éventuellement un à trois substituants, identiques ou différents, alkyle en C₁ à C₄ ou halogéno, ainsi que phényle, phénoxy, phénylthio ou benzyle portant chacun, le cas échéant, un à trois substituants, identiques ou différents, halogéno, nitro, cyano, alkyle en C₁ à C₄, halogénalkyle en C₁ à C₄ ayant 1 à 5 atomes d'halogènes identiques ou différents tels que des atomes de fluor et de chlore, alkoxy en C₁ à C₄, halogénalkoxy en C₁ à C₄ ayant 1 à 5 atomes d'halogènes identiques ou différents tels que des atomes de fluor et de chlore, alkylthio en C₁ à C₄, halogénalkylthio en C₁ à C₄ ayant 1 à 5 atomes d'halogènes identiques ou différents tels que des atomes de fluor et de chlore, (alkylthio en C₁ à C₄)-(alkyle en C₁ à C₄), alkylsulfonyle en C₁ à C₄, halogénalkylsulfonyle en C₁ à C₄ ayant 1 à 5 atomes d'halogènes identiques ou différents tels que des atomes de fluor et de chlore, ou (alkoxy en C₁ à C₄)-carbonyle,
ou bien un reste cycloalkyle en C₃ à C₈ ou cyclo-alcényle en C₅ à C₉ portant chacun, le cas échéant, un à trois substituants identiques ou différents, les substituants considérés étant des substituants alkyle en C₁ à C₄, halogénalkyle en C₁ à C₄ ayant 1 à 5 atomes d'halogènes identiques ou différents tels que des atomes de fluor et de chlore, alkoxy en C₁ à C₄, halogénalkoxy en C₁ à C₄ ayant 1 à 5 atomes d'halogènes identiques ou différents tels que des atomes de fluor et de chlore, cycloalkyle en C₃ à C₈, ainsi que phényle portant éventuellement un à trois substituants, identiques ou différents, halogéno, cyano, nitro, alkyle en C₁ à C₄, halogénalkyle en C₁ à C₄ ayant 1 à 5 atomes d'halogènes identiques ou différents tels que des atomes de fluor et de chlore, alkoxy en C₁ à C₄ ou halogénalkoxy en C₁ à C₄ ayant 1 à 5 atomes d'halogènes identiques ou différents tels que des atomes de fluor et de chlore, et
Y est un reste alkylène en C₁ à C₆, hydroxyalkylène en C₁ à C₆, (alkoxy en C₁ à C₄)-(alkylène en C₁ à C₆), (alkyle en C₁ à C₄)carbonyloxy-(alkylène en C₁ à C₆), cyano-(alkylène en C₁ à C₆), halogénalkylène en C₁ à C₄ ayant 1 à 5 atomes d'halogènes identiques ou différents tels que des atomes de fluor et de chlore ; (cycloalkyle en C₃ à C₆)-alkylène en C₁ à C₄, alcénylène en C₂ à C₄ ou alkylèneoxy en C₁ à C₄ portant éventuellement un à trois substituants, identiques ou différents, fluoro, chloro ou méthyle.

2. Composés de formule (I) suivant la revendication 1, formule dans laquelle
R¹ est un reste méthyle, éthyle, n-propyle, isopropyle, n-butyle, isobutyle, sec.-butyle, tertio-butyle ; CH₂F, CHF₂, CF₃, CH₂Cl, CH₂Br, CHClCH₃ ; méthoxy, éthoxy, méthoxyméthyle, éthoxyméthyle ; méthylthiométhyle ou cyclopropyle,
R² est l'hydrogène, un reste méthyle, éthyle, n-propyle, isopropyle ; méthoxyméthyle, éthoxyméthyle, n-propoxyméthyle, n-butoxyméthyle ; méthylcarbonyle, méthylsulfonyle ; phénylcarbonyle ou benzyle portant chacun, le cas échéant, un ou deux substituants, identiques ou différents, fluoro, chloro, méthyle ou trifluorométhyle ; ou cyclopropyle,
R³ est un reste phényle portant éventuellement un à trois substituants identiques ou différents, les substituants considérés étant des substituants halogéno, nitro, cyano, alkyle en C₁ à C₁₂, halogénalkyle en C₁ à C₁₂ ayant 1 à 5 atomes d'halogènes identiques ou différents tels que des atomes de fluor et de chlore, alkoxy en C₁ à C₆, halogénalkoxy en C₁ à C₄ ayant 1 à 5 atomes d'halogènes identiques ou différents tels que des atomes de fluor et de chlore, alkylthio en C₁ à C₄, halogénalkylthio en C₁ à C₄ ayant 1 à 5 atomes d'halogènes identiques ou différents tels que des atomes de fluor et de chlore, alcényle en C₂ à C₄, halogénalcényle en C₂ à C₄ ayant 1 à 5 atomes d'halogènes identiques ou différents tels que des atomes de fluor et de chlore, ainsi que phénoxy ou phénylthio portant chacun, le cas échéant, un à trois substituants, identiques ou différents, halogéno, nitro, cyano, alkyle en C₁ à C₄, halogénalkyle en C₁ à C₄ ayant 1 à 5 atomes d'halogènes identiques ou différents tels que des atomes de fluor et de chlore, alkoxy en C₁ à C₄, halogénalkoxy en C₁ à C₄ ayant 1 à 5 atomes d'halogènes identiques ou différents tels que des atomes de fluor et de chlore, alkylthio en C₁ à C₄, halogénalkylthio en C₁ à C₄ ayant 1 à 5 atomes d'halogènes identiques ou différents tels que des atomes de fluor ou de chlore, (alkylthio en C₁ à C₄)-(alkyle en C₁ à C₄), alkylsulfonyle en C₁ à C₄, halogénalkylsulfonyle en C₁ à C₄ ayant 1 à 5 atomes d'halogènes identiques ou différents tels que des atomes de fluor et de chlore, ou (alkoxy en C₁ à C₄)-carbonyle et
Y représente l'un des groupes
-CH₂-, -CH(CH₃)-, -CH(C₂H₅)-, -CH(n-C₃H₇)-, -CH(i-C₃H₇)-, -CH₂CH₂-, -CH(OH)-, -CH(OCH₃)-, -CH(C-CO-CCH₃)-, -CH(CN)-, -CHF-, -CHCl-, -CH-[ 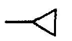 ]-, -CH=CH- ou -CH₂O-

3. Composés de formule (I) suivant la revendication 1, formule dans laquelle
R¹ est un reste méthyle, éthyle, n-propyle, iscpropyle, n-butyle, isobutyle, sec.-butyle ou tertio-butyle ; CH₂F, CHF₂, CF₃, CH₂Cl, CH₂Br, méthoxy, éthoxy ou cyclopropyle,
R² est l'hydrogène, un reste méthyle, éthyle, méthoxyméthyle, éthoxyméthyle, méthylcarbonyle, phénylcarbonyle ou méthylsulfonyle,
R³ est un reste phényle portant éventuellement un à trois substituants identiques ou différents, les substituants considérés étant des substituants fluoro, chloro, bromo, nitro, cyano, méthyle, éthyle, n-propyle, isopropyle, n-butyle, isobutyle, sec.-butyle ou tertio-butyle ; méthoxy, éthoxy, n-propoxy, isopropoxy, n-butoxy, isobutoxy, sec.-butoxy ou tertiobutoxy ; méthylthio, CF₃, OCF₃, OCHF₂, SCF₃, SCCl₂F, CH₂Br, CH₂Cl ainsi que phénoxy portant éventuellement un à trois substituants, identiques ou différents, fluoro, chloro, bromo, nitro, cyano, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, méthylthiométhyle, CF₃, OCF₃, OCHF₂, SCF₃, SCOl₂F, CH₂Br, CH₂Cl, méthoxycarbonyle, méthylsulfonyle ou trifluorométhylsulfonyle et
Y est un reste -CH₂-, -CH(CH₃)-, -CH₂CH₂- ou -CH=CH-.

4. Procédé de production de composés de formule (I) suivant la revendication 1, **caractérisé en ce qu'**on fait réagir des 5-amino-1,2,4-thiadiazoles de formule (II) 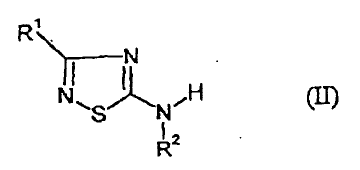 dans laquelle
R¹ et R² ont la définition indiquée dans la revendication 1,
avec des halogénures d'acides de formule (III)
Hal―CO―Y―R³ (III)
dans laquelle
R³ et Y ont la définition indiquée dans la revendication 1 et
Hal est un halogène,
en présence d'une base et en présence d'un diluant.

5. Compositions pesticides, **caractérisées par** une teneur en au moins un composé de formule (I) suivant la revendication 1.

6. Utilisation de composés de formule (I) suivant la revendication 1 pour combattre des parasites.

7. Procédé pour combattre des parasites, **caractérisé en ce qu'**on fait agir des composés de formule (I) suivant la revendication 1 sur les parasites et/ou sur leur milieu.

8. Procédé de préparation de compositions pesticides, **caractérisé en ce qu'**on mélange des composés de formule (I) suivant la revendication 1 avec des diluants et/ou des agents tensio-actifs.

9. Utilisation de composés de formule (I) suivant la revendication 1 pour la préparation de compositions pesticides.
